(19) Europäisches Patentamt / European Patent Office / Office européen des brevets

(11) **EP 2 013 616 B1**

(12) **EUROPÄISCHE PATENTSCHRIFT**

(45) Veröffentlichungstag und Bekanntmachung des Hinweises auf die Patenterteilung:
**21.12.2011   Patentblatt 2011/51**

(21) Anmeldenummer: **07724549.6**

(22) Anmeldetag: **25.04.2007**

(51) Int Cl.:
*G01N 33/20* (2006.01)     *G01N 7/00* (2006.01)

(86) Internationale Anmeldenummer:
**PCT/EP2007/003619**

(87) Internationale Veröffentlichungsnummer:
**WO 2007/121994 (01.11.2007 Gazette 2007/44)**

(54) **VERFAHREN ZUM NACHWEIS VON WASSERSTOFF IN STAHL**

METHOD FOR THE DETECTION OF HYDROGEN IN STEEL

PROCÉDÉ DE DÉTECTION D'HYDROGENE DANS D'ACIER

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **25.04.2006   DE 102006020426**

(43) Veröffentlichungstag der Anmeldung:
**14.01.2009   Patentblatt 2009/03**

(73) Patentinhaber:
• **Georg-August-Universität Göttingen Stiftung des öffentlichen Rechts (ohne Bereich Humanmedizin)**
**37073 Göttingen (DE)**
Benannte Vertragsstaaten:
**DE**
• **AB SKF**
**415 50 Göteborg (SE)**
Benannte Vertragsstaaten:
**DE**
• **Kirchheim, Reiner**
**37075 Göttingen (DE)**
Benannte Vertragsstaaten:
**AT BE BG CH CY CZ DK EE ES FI FR GB GR HU IE IS IT LI LT LU LV MC MT NL PL PT RO SE SI SK TR**

(72) Erfinder:
• **KIRCHHEIM, Reiner**
**37075 Göttingen (DE)**
• **GEGNER, Jürgen**
**90765 Fürth (DE)**
• **WILBRANDT, Peter-Joachim**
**37085 Göttingen (DE)**

(74) Vertreter: **Patentanwälte**
**Ruff, Wilhelm, Beier, Dauster & Partner**
**Postfach 10 40 36**
**70035 Stuttgart (DE)**

(56) Entgegenhaltungen:
**EP-A- 0 015 428     WO-A-03/093530**
**US-A- 3 732 076     US-A- 3 909 617**

• **C. BÉGUIN ET AL.: "Tantalum Coating of Mild Steel by Chemical Vapour Deposition" THIN SOLID FILMS, Bd. 46, 1977, Seiten 209-212, XP002442115**

**Beschreibung**

Die Erfindung betrifft in erster Linie ein Verfahren zum Nachweis von Wasserstoff in Stahl.

Es ist eine bekannte Tatsache, daß bestimmte unerwünschte Gehalte an Fremdstoffen in anderen Materialien, insbesondere in metallischen Werkstoffen, negative Auswirkungen auf die physikalischen oder chemischen Eigenschaften dieser Materialien haben können. So ist es in bestimmten Fällen unerwünscht, daß in einem Metall oder einer Metallegierung bestimmte Gehalte an anderen Metallen enthalten sind. Ein Beispiel ist hier der nicht akzeptable Bleigehalt in Kupferlegierungen, die zur Herstellung von wasserführenden Bauteilen im Sanitärbereich eingesetzt werden sollen.

In besonderem Maße stellt sich die oben genannte Problematik bei gasförmigen Verunreinigungen, da die betreffenden Atome in den verschiedenen Materialien, insbesondere in metallischen Werkstoffen, zum einen in vergleichsweise hohen Konzentrationen löslich und zum anderen in diesen Materialien in vielen Fällen hochbeweglich sind. Solche gasförmigen Verunreinigungen können jedoch auf die Materialeigenschaften einen großen, häufig unerwünschten Einfluß ausüben.

Von den gasförmigen Verunreinigungen wiederum ist insbesondere der Wasserstoff hervorzuheben. Dessen starker negativer Einfluß auf Materialien, insbesondere auf metallische Werkstoffe, ist seit langem unter dem Begriff der Wasserstoffversprödung bekannt. Insbesondere bei Stählen und den aus Stahl gefertigten Bauteilen muß durch den Einfluß von Wasserstoff häufig mit einer deutlichen Beeinträchtigung des Betriebsverhaltens gerechnet werden.

Gerade bei Stählen tritt die unerwünschte Aufnahme von Wasserstoff sowohl bei deren Herstellung als auch bei deren Veredelung und bei ihrem bestimmungsgemäßen Einsatz auf. Beispielhaft sind hier die Verfahrensschritte des Gießens, Schweißens, Beizens oder Galvanisierens zu nennen (Hoffmann, F.; Linkewitz, T.; Mayr, P.: Wasserstoffversprödung - Meinungen und Fakten. In: Einsatzhärtung. Berichtsband der AWT-ATTT-Tagung, 29.-30. April 1998, Aachen, Arbeitsgemeinschaft Wärmebehandlung und Werkstofftechnik e.V. (AWT)/Association Technique de Traitement Thermique (ATTT), Wiesbaden (1998) S. 259-267; Hoffmann, F.; Linkewitz, T.; Mayr, P.: Wasserstoffaufnahme beim Einsatzhärten. HTM 54 (1999) 10-13).

Weiter ist bei der industriellen Aufkohlung, insbesondere aus der Gasphase, der Eintrag von Wasserstoff in das wärmebehandelte Stahlbauteil verhältnismäßig hoch (Wyss, U.: Aufkohlen in Gasen: In: Benninghoff, H. (Hrsg.): Wärmebehandlung der Bau- und Werkzeugstähle. BAZ Verlag, Basel (1978)). Bei diesen Verfahren entsteht der Wasserstoff bei den primären Spaltreaktionen der Aufkohlungsmittel zur Erzeugung der Gasatmosphäre und über die Oberflächenreaktionen, die Kohlenstoff freisetzen. Auch bei dem sogenannten Direkthärten kann ein Anteil von bis zu 2,2 Gew.-ppm Wasserstoff (entspricht 122,1 at.-ppm) resultieren (Streng, H.; Grosch, J.; Razim, C.: Wasserstoffaufnahme und -abgabe beim Einsatzhärten. HTM (Härterei-Technische Mitteilungen) 42 (1987) 245-260). Wegen der hohen Beweglichkeit der Wasserstoffatome im Eisengitter des Stahls genügt die übliche Aufkohlungszeit zur Verteilung des Wasserstoffs über das gesamte Volumen des Werkstücks. Deshalb treten die durch Wasserstoff verursachten typischen Fischaugen an Einschlüssen auch im Inneren von dicken Bauteilen auf.

Darüber hinaus kann Wasserstoff auch über Korrosion metallischer Legierungen in wasserstoffhaltigen Medien gebildet werden und in den Werkstoff eindringen. So wird etwa Wasserstoff im Betrieb geschmierter Maschinenelemente über tribochemische Reaktionen mit dem Schmierstoff von der Oberfläche her in das Stahlbauteil eingebracht.

Die Erscheinungsformen der Wasserstoffversprödung und die dazu führenden Vorgänge im Material werden schon seit langem untersucht und diskutiert. So wurde versucht, die durch Wasserstoff hervorgerufene Werkstoffschädigung mit vier prinzipiellen Mechanismen zu erklären (Neumann, P.: Grundlagen der Wirkung von Wasserstoff auf die Rißbildung in Stählen. Stahl u. Eisen 107 (1987) 577-583). Diese vier Mechanismen sind:

-    Bildung spröder Carbide an Rißspitzen aufgrund der Mehrachsigkeit des Spannungszustands.
-    innere Methanbildung durch Reaktion des Wasserstoffs mit gelöstem Kohlenstoff (innere Entkohlung).
-    direkte mechanische Schädigung durch hohe Wasserstoff-Gleichgewichtsdrucke (Rekombination der gelösten Wasserstoffatome zu Wasserstoff-Molekülen, bevorzugt an Fehlstellen).
-    Minderung der atomaren Kohäsionskräfte durch den Wasserstoff, verbunden mit starker Sprödbruchneigung.

Bei den gasförmigen Verunreinigungen, insbesondere bei der geschilderten Wasserstoffversprödung, ist nicht nur problematisch, daß die Werkstoffschädigung überhaupt entsteht, sondern auch, daß sich der Einfluß dieser gasförmigen Verunreinigungen häufig nicht mit ausreichender Empfindlichkeit nachweisen und untersuchen läßt. So ist es im Falle von Wasserstoff zwar grundsätzlich möglich, diesen qualitativ nachzuweisen. Für die quantitativen Messungen, die für ein fundiertes Verständnis der zugrunde liegenden Vorgänge notwendig sind, stehen jedoch entweder keine ausreichend empfindlichen Methoden zur Verfügung, oder die zur Verfügung stehenden Methoden lassen sich im entsprechenden Umfeld bei der Herstellung oder beim Einsatz der betreffenden Materialien nicht oder nicht zufriedenstellend anwenden.

Gerade eine quantitative Bestimmung ist auch aus dem im Stand der Technik bekannten US-Patent 3,732,076 nicht ersichtlich. Dort wird der Nachweis von Wasserstoff lediglich anhand mikroskopischer und elektronenmikroskopischer Beobachtungen geführt. Aufgrund der so erhaltenen ungenauen Ausgangsdaten lassen sich keine reproduzierbaren quantitativen Bestimmungen der Wasserstoffkonzentration in der dort verwendeten Probe durchführen.

In diesem Zusammenhang ist es aus der Veröffentlichung von R. Kirchheim, Acta Metallurgica, Vol. 27, pp 869 bis 878 (1979) bekannt, die Diffusionskoeffizienten von Sauerstoff in Kupfer, Niob und Tantal mit Hilfe von Metallen, in denen Sauerstoff eine größere Löslichkeit besitzt, zu bestimmen. In dieser fast 30 Jahre alten Veröffentlichung ist aber weder die Problematik von gasförmigen Verunreinigungen in metallischen Werkstoffen noch deren Nachweis angesprochen. Auch die Thematik der Wasserstoffversprödung ist nirgends erwähnt.

Die EP-A1 0 015 428 offenbart ein Verfahren sowie eine Vorrichtung zur Abtrennung von Wasserstoff aus Fluiden. Zu diesem Zweck wird das Fluid mit Titan-Zirkon-Legierungen, die mit Nickel, Kobalt, Eisen, Palladium, Platin, Vanadium, Niob oder Tantal oder deren Legierungen oberflächenbeschichtet sind, in Kontakt gebracht. Der im Fluid enthaltene Wasserstoff durchdringt dann die beschichtete Legierung und kann entweder in der Legierung gespeichert oder aus ihr kontinuierlich oder zeitweise entfernt werden. Auf diese Weise wird der Wasserstoff vom Fluid, insbesondere von flüssigem Natrium abgedrängt.

Weder das Material Stahl noch eine Bestimmung der Konzentration des Wasserstoffs sind in dieser Druckschrift erwähnt.

Dementsprechend stellt sich die vorliegende Erfindung die Aufgabe, Wasserstoff in Stahl mit hoher Empfindlichkeit nachzuweisen. Dabei soll eine quantitative Bestimmung solcher Verunreinigungen möglich werden.

Diese Aufgabe wird gelöst durch das erfahren nach Ansprüch 1. Bevorzugte Ausführungen des beanspruchten Verfahrens sind in den abhängigen Ansprüchen 2 bis 8 dargestellt. Der Wortlaut sämtlicher Ansprüche wird hiermit durch Bezugnahme zum Inhalt dieser Beschreibung gemacht.

Die Erfindung umfaßt ein Verfahren der eingangs genannten Art, bei dem der im Stahl als erstern Material Vorhandene Wasserstoff mindestens teilweise in Vanadium, Niob oder Tantal oder deren Legierungen als zweites Material überführt wird. Dieser Verfahrensschritt erfolgt vorzugsweise unter Anreicherung des Wasserstoffs in dem zweiten Material.

Weiter wird bei dem erfindungsgemäßen Verfahren se die Konzentration von Wassersstoff im zweiten Material quantitativ bestimmt.

Daraus wird bei dem erfindungsgemäßen Verfahren die Konzentration von Wasserstoff im ersten Material quantitativ bestimmt.

Das geschilderte erfindungsgemäße Verfahren läßt sich auch so beschreiben, daß der Wasserstoff aus dem ersten Material in ein zweites Material, in dem sich der Wasserstoff leichter oder besser, quantifizieren läßt, überführt wird. Der eigentliche (direkte) Nachwels des Wasserstoffs erfolgt dann in diesem zweiten Material, und aus diesem Nachweis im zweiten Material werden Rückschlüsse auf die Anwesenheit der Verunreinigungen im ersten Material gezogen.

Aus den bisherigen Ausführungen ergibt sich, daß das zweite Material aufgrund seiner größeren Affinität für Wasserstoff als sogenannte Senke für die Verunreinigung Wasserstoff wirkt. Man kann dies auch so beschreiben, daß das zweite Material den Wasserstoff wie eine Art Staubsauger oder Absorber in sich hineinsaugt. Bei gasförmigen Verunreinigungen wie Wasserstoff erfolgt dieser Übergang aus dem ersten Material in das zweite Material in relativ kurzer Zeit. Dies läßt sich für Wasserstoff beispielsweise wie folgt abschätzen:

Es soll von einer 200 $\mu$m dicken Probe aus einem ersten Material ausgegangen werden, auf der sich eine 20 nm dicke Oberflächenschicht aus einem zweiten Material befindet. In der Probe aus erstem Material sei eine Wasserstoffkonzentration von 1 Gew.-ppm enthalten. Dies ist eine typische Größenordnung für einen technischen Stahl und entspricht etwa 55,5 at.-ppm. Wird der gesamte in der Probe enthaltene Wasserstoff in die Oberflächenschicht aus dem zweiten Material überführt, so erhöht sich dort die Wasserstoffkonzentration theoretisch auf 55,5 at.-% Wasserstoff (bei angenommener gleicher Gitterkonstante). Nimmt man einen typischen Diffusionskoeffizienten für die Wasserstoffdiffusion in ferritischen Eisenlegierungen von $D=1E-6cm^2/s$ an, so erfolgt bei ungestörter Diffusion die Anreicherung des Wasserstoffs im zweiten Material in einer Zeit von ca. 100 Sekunden.

Diese Plausibilitätsbetrachtung zeigt für Wasserstoff nicht nur, daß die Überführung aus dem ersten Material in das zweite Material in relativ kurzer Zeit erfolgt, sondern auch, daß bei bekannter Dicke der Schicht aus dem zweiten Material und bei bekannten Diffusionsdaten für Wasserstoff in der Probe aus erstem Material ein direkter Rückschluß auf die Wasserstoffkonzentration in der Probe möglich ist, und zwar über eine quantitative Bestimmung des Wasserstoffs im zweiten Material. Es besteht ein direkter Zusammenhang zwischen der Wasserstoffkonzentration in der Schicht aus dem zweiten Material und der Wasserstoffkonzentration in den darunterliegenden Gefügebereichen der Probe aus dem ersten Material.

Dies wird in der nun folgenden Beschreibung und auch im Zusammenhang mit dem Beispiel noch näher erläutert.

Wie bereits erwähnt erfolgt bei dem erfindungsgemäßen Verfahren die Überführung des Wasserstoffs aus dem ersten Material in das zweite Material durch Diffusion. Diese Diffusion wird im Falle von Wasserstoff, schon bei Raumtemperatur ausreichend sein.

Die Überführung des Wasserstoffs aus dem ersten Material in das zweite Material kann bei der Erfindung insbesondere auch unter Anlegen eines elektrischen Felds (gegebenenfalls auch unter Anlegen eines Magnetfelds, alternativ oder zusätzlich) erfolgen. Mit derartigen Maßnahmen kann die Diffusion des Wasserstoffs aus dem ersten Material in das zweite Material initiiert oder unterstützt werden.

Um eine ungestörte Überführung/Anreicherung des Wasserstoffs aus dem ersten Material in das zweite Material zu gewährleisten, muß das erste Material mit dem zweiten Material in ausreichendem Kontakt stehen. Dazu kann es genügen, wenn einzelne Bereiche dieser beiden Materialien miteinander in Kontakt, vorzugsweise in innigem Kontakt, stehen. Vorzugsweise stehen dabei die beiden Materialien über ihre gesamten Oberflächenbereiche miteinander in Kontakt, insbesondere in innigem Kontakt. Auf diese Weise läßt sich des Wassertoff möglichst rasch und vollständig aus dem ersten Material in das zweite Material überführen.

Bei der Erfindung hat das zweite Material eine wesentlich höhere Affinität für die Verunreinigung als das erste Material. Auf diese Weise können, im Vergleich zum Volumen des ersten Materials, kleine Volumina an zweitem Material vorgesehen und ausreichend sein. Dies ist auch deshalb von Vorteil, da sich in kleineren Volumina des zweiten Materials größere Konzentrationen an Verunreinigung (und damit größere Anreicherungen der Verunreinigung) erreichen lassen. Dies erleichtert den quantitativen, Nachweis des Wasserstoffs im zweiten Material.

In Übereinstimmung mit den zuletzt gemachten Ausführungen ist es dementsprechend bei der Erfindung bevorzugt, wenn das zweite Material als Schicht, insbesondere als dünne Schicht auf ein Substrat oder eine Probe aus dem ersten Material aufgebracht ist. Dabei befindet sich diese Schicht zumindest auf einem Teil der Oberflächen dieses Substrats oder dieser Probe, vorzugsweise auf der gesamten Oberfläche dieses Substrats oder dieser Probe.

Die Abmessungen, z. B. die Dicke des Substrats oder der Probe, sind bei der Erfindung grundsätzlich nicht kritisch. Wie oben abgeleitet, finden, im Falle von Wasserstoff als Verunreinigung, auch bei dickeren Proben, die entsprechenden Diffusionen in ausreichend kurzer Zeit statt. In solchen Fällen sind auch andere Geometrien als die einer dünnen Schicht für das zweite Material denkbar. So kann beispielsweise das zweite Material in länglicher bzw. langgestreckter Form, insbesondere als Band, Stift, Draht oder dergleichen, beispielsweise ein Vanadiumdraht mit dem ersten Material in Kontakt gebracht werden, beispielsweise durch Verschweißen, insbesondere Reibverschweißen, und die beispielsweise vom Vanadium aufgenommene Wasserstoffmenge leicht über die Änderung des elektrischen Widerstandes verfolgt werden.

Beim Reibverschweißen bzw. Reibschweißen handelt es sich bekanntlich um ein Schweißverfahren, bei dem zwei Teile relativ zueinander bewegt werden, wobei sich die Teile an den Kontaktflächen berühren. Durch die so entstehende Reibung kommt es zu einer Erwärmung. Am Ende des Reibvorganges werden die Teile entsprechend zueinander positioniert und unter Druck, in der Regel unter hohem Druck, miteinander verbunden. Der Vorteil des Reibverschweißens liegt unter anderem darin, dass ein deutlich kleinerer Bereich der entsprechenden Teile erwärmt wird als bei anderen Schweißverfahren und dass es in der Regel nicht zur Bildung einer Schmelze in der Zone kommt, in der die Teile miteinander verfügt werden.

Im Falle einer Schichtgeometrie beträgt in den oben beschriebenen Fällen die Schichtdicke des zweiten Materials vorzugsweise weniger als 1 $\mu$m, vorzugsweise weniger als 250 nm. Desweiteren sind Schichtdicken des zweiten Materials zwischen 10 und 100 nm weiter bevorzugt. Insbesondere können Schichtdicken des zweiten Materials von vorzugsweise ca. 20 nm gewählt werden. Bei den üblichen Abmessungen entsprechender Substrate/Proben sind die auf diese Weise erhaltenen Anreicherungen von Wasserstoff in der Schicht ausreichend.

Die Volumina des zweiten Materials, insbesondere die beschriebenen Schichten aus dem zweiten Material, können grundsätzlich mit den unterschiedlichsten Verfahren auf die entsprechenden Substrate oder Proben aus dem ersten Material aufgebracht werden. Vorzugsweise sind hier die sogenannten CVD (chemical vapor deposition)-Verfahren und insbesondere die PVD (physical vapor deposition)-Verfahren zu nennen. CVD-Verfahren nutzen die chemische Abscheidung von Schichten aus der Gasphase. Dabei wird an der erhitzten Oberfläche eines Substrats aufgrund einer chemischen Reaktion aus der Gasphase eine Feststoffkomponente abgeschieden. Unter PVD-Verfahren faßt man eine Gruppe von Beschichtungsverfahren im Vakuum zusammen, bei denen die Schicht durch Kondensation eines Materialdampfes gebildet wird. Häufig wird dabei auf ein sogenanntes Target aus dem aufzutragenden Material ein Ionenstrahl oder Laserstrahl gerichtet und dieses Material dabei zerstäubt. Dieses zerstäubte Material schlägt sich dann auf der Oberfläche des Substrats nieder.

Soll das zweite Material als dickere Schicht oder in einer anderen Form mit dem ersten Material verbunden werden, so kann durch plastische Verformung der Kontaktbereiche (z. B. über Reibverschweißen, Explosionplattieren, Pulverspritzen) oder durch thermisches Verschweißen ein unmittelbarer inniger Verbund erreicht werden. Im Falle des Wasserstoffes ist dabei insbesondere die vollständige oder teilweise Beseitigung der gegebenenfalls vorhandenen natürlichen Oxidhäute der ersten und zweiten Materialien zweckmäßig oder erforderlich, da diese den Wasserstoffübertritt zwischen beiden Materialien unterbinden oder zumindest stark hemmen können.

Bei dem erfindungsgemäßen Verfahren kann die Anwesenheit des Wasserstoffs in dem zweiten Material ebenfalls mit den unterschiedlichsten Methoden detektiert und quantitativ bestimmt werden. Hier sind beispielsweise naßchemische Bestimmungen möglich oder die sogenannte Thermodesorption (TDS). Ein bevorzugter Nachweis erfolgt beispielsweise über die sogenannte Röntgendiffraktometrie (Röntgenbeugung), mit deren Hilfe sich beispielsweise die Änderung der Gitterkonstante des entsprechenden Materials durch die Anreicherung des Wasserstoffs in diesem Material ermitteln läßt. So führt beispielsweise im Material Niob eine Erhöhung der Wasserstoffkonzentration von 0 auf 1 at.-% zu einer relativen Änderung der Gitterkonstante von 5x10E-4. Solche Änderungen der Gitterkonstante lassen sich mit modernen Röntgendiffraktometern nachweisen. Die maximale Löslichkeit von Wasserstoff im Niob beträgt ca. 6 at.-% bei 295 K, und es bildet sich bei höheren Konzentrationen eine zweite Phase mit den zugehörigen Bragg-Reflexen. Aus den Intensitätsverhältnissen von Hydridphase und $\alpha$-Phase können deren Volumenanteile berechnet werden und damit der gesamte im Niob absorbierte Gehalt an Wasserstoff.

Eine ebenfalls besonders bevorzugte Nachweismethode ist die Messung des elektrischen Widerstandes, die sich für den Fall von kontaktierten Drähten, Stiften oder Bändern des zweiten Materials besonders eignet. So bewirkt eine Erhöhung der Wasserstoffkonzentration um 0,1 at.-% in Vanadium einen Anstieg des spezifischen Widerstandes von 0,01 $\mu$Ohm cm. Diese und noch wesentlich kleinere Änderungen lassen sich direkt oder, noch empfindlicher, als Differenzmessung zu einer nicht kontaktierten Vanadiumprobe gleicher Geometrie bestimmen.

Eine weitere bei der Erfindung bevorzugte Methode zum Nachweis des Wasserstoffs im zweiten Material ist die elektrochemische Methode, bei dem die beiden Materialien (erstes und zweites Material) Teile einer galvanischen Zelle bilden. Die Probe aus erstem Material und die darauf sich befindende Schicht aus zweitem Material bilden dabei die Arbeitselektrode der galvanischen Zelle und werden mit einer Referenz- und Gegenelektrode unter Ausbildung eines geeigneten Potentials für einen elektrochemischen Wasserstoffentzug zusammengeschaltet.

Die genaue Auswertung der Daten, die durch die Überführung/Anreicherung des Wasserstoffs im zweiten Material erhalten werden, wird später im Zusammenhang mit dem Beispiel noch näher erläutert. An dieser Stelle sei vorab lediglich erwähnt, daß es bei der Erfindung bevorzugt ist, die (an der Kontaktfläche zwischen erstem Material und zweiten Material) pro Flächeneinheit vom zweiten Material aufgenommene Menge an Wasserstoff als Funktion der Zeit zu berechnen und diese auf die Flächeneinheit bezogene Menge al Kennwert für die Eigenschaften des ersten Materials im Hinblick auf den Wasserstoff auszuwählen. Im Falle von Wasserstoff als Verunreinigung dient dieser Kennwert dann als Kennwert für die Neigung des ersten Materials zur Wasserstoffversprödung.

Die im Beispiel enthaltenen Erläuterungen zeigen auch, daß das erfindungsgemäße Verfahren Aussagen ermöglicht, wie stark der Wasserstoff im ersten Material gebunden ist. In diesem Zusammenhang ist es bei bevorzugten Ausführungsformen des erfindungsgemäßen Verfahrens vorgesehen, den Wasserstoff, in mindestens zwei, vorzugsweise mehrere zweite Materialien zu überführen und daraus gegebenenfalls die Bindungsstärke des Wasserstoffs im ersten Material abzuleiten. Je nach Affinität des zweiten Materials zum Wasserstoff kann dann der Anteil der verschieden stark gebundenen Atome oder Moleküle der Wasserstoffatome im ersten Material bestimmt werden. Auch dies wird im Zusammenhang mit dem Beispiel noch näher erläutert.

Weitere Merkmale der Erfindung ergeben sich aus der nachfolgenden Beschreibung einer bevorzugten Ausführungsform in Verbindung mit den Unteransprüchen. Hierbei können die einzelnen Merkmale jeweils für sich allein oder zu mehreren in Kombination miteinander verwirklicht sein. Das beschriebene Beispiel dient lediglich der Erläuterung und zum besseren Verständnis der Erfindung und ist in keiner Weise einschränkend zu verstehen.

**Beispiel 1**

Auf ein dünnes Blech (Dicke d=1 mm) aus einem Baustahl, dessen Wasserstoffgehalt nach dem Heißextraktionsverfahren zu ca. (0,5 $\pm$ 0,1) Gew.-ppm bestimmt wurde, wurde unter Ultrahochvakuumbedingungen die natürliche Oxidhaut durch Ionenätzen (Sputtern) entfernt und mit Hilfe eines PVD-Verfahrens eine Niobschicht in einer Dicke von ca. 200 nm aufgebracht. Das Molvolumen der Niobschicht $V_a$ ($_a$ steht für absorber) beträgt 10,8 cm$^3$/mol. Die Niobschicht wurde mit einer 5 nm dicken Palladiumschicht abgedeckt. So wird die Niobschicht vor Oxidation geschützt und eine elektrochemische Bestimmung des Wasserstoffs ermöglicht.

Die Ausgangskonzentration $c_o$ an Wasserstoff im Stahl von 0,5 Gew.-ppm entspricht 3,9 E-6 Mol-H/cm$^3$. Die Diffusionskonstante D des Wasserstoffs im verwendeten Stahl wird mit D=1E-6cm$^2$/s angenommen. Die weitgehende Absorption des Wasserstoffs in die Niobschicht hinein erfolgt innerhalb einer Zeit von ca. d$^2$/D = 1E4 s.

Anschließend wird nach einer Wartezeit von 4 Stunden in einer Röntgenbeugungsmessung der Reflex der Mischkristallphase des Niobs mit einem Gehalt von Mol-H/Mol-Nb = 0,06 und der Reflex der Niobhydridphase mit Mol-H/Mol-Nb = 1 detektiert. Aus dem Verhältnis der Flächen unter beiden Peaks errechnet sich der gesamte Wasserstoffgehalt in der Niobschicht zu Mol-H/Mol-Nb = 0,20 oder 1,8E-2 Mol-H/cm$^3$.

Rechnerisch kann das durchgeführte Experiment wie folgt dargestellt werden:

Im vorliegenden Fall wird davon ausgegangen, daß die gesamte, im Stahl enthaltene Wasserstoffmenge in die Niobschicht überführt und dort angereichert wird. Wie bereits ausgeführt, werden bei der Erfindung die Volumina der zu untersuchenden Probe $V_P$ (hier technischer Stahl als erstes Material) und des Absorbers $V_A$ (hier Niob als zweites Material) mit Vorteil so dimensioniert, daß die Aufnahmefähigkeit des Absorbers für Wasserstoff nicht überschritten wird.

Dementsprechend ist die Menge an Wasserstoff, die aus der Probe abgegeben wird, gleich der Menge an Wasserstoff, die vom Absorber aufgenommen wird. Die Wasserstoffkonzentration in der Probe berechnet sich dann aus der im Absorber gemessenen Wasserstoffkonzentration zu:

$$c_0 = \frac{V_P}{V_A} c_A$$

mit

$c_0$ = Wasserstoffkonzentration in der Probe in Mol-H pro $cm^3$, und
$C_A$ = Wasserstoffkonzentration im Absorber in Mol-H pro $cm^3$.

Für den im Experiment realisierten Fall, daß die Grundflächen beider Materialien gleich sind vereinfacht sich die letzte Gleichung zu

$$c_0 = \frac{d_P}{d_A} c_A \ ,$$

in der d für die Dicke des jeweiligen Materials steht. Mit dem experimentellen Wert von $c_0$ = 1,8E-2 Mol-H/$cm^3$ ergibt sich somit in guter Übereinstimmung mit dem Heißextraktionswert $c_A$ zu 3,6E-2 Mol-H/$cm^3$ oder 0,46 Gew.-ppm.

Bei den wichtigen Anwendungsfällen der vorliegenden Erfindung ist das Volumen der Probe $V_P$ in der Regel groß oder sehr groß gegenüber dem Volumen des Absorbers $V_A$. Dies sind die Fälle, in denen es sich bei der Probe um ein vorgegebenes Bauteil handelt, das in seinen Dimensionen selbstverständlich nicht veränderbar ist. In diesen Fällen kann man aus der im Absorber gemessenen Wasserstoffkonzentration $C_A$ ein Maß für die Gesamtkonzentration und Beweglichkeit des Wasserstoffs in der Probe ableiten. Dies geschieht wie folgt.

Vor Beginn des Kontaktes zwischen Probenmaterial (erstes Material) und Absorber (zweites Material) bzw. vor Übertritt des Wasserstoffs aus der Probe in den Absorber (bei bereits kontaktiertem Probenmaterial und Absorber) ist die Wasserstoffkonzentration in der Probe überall gleich der Konzentration $C_0$. Ein (ideal gewählter) Absorber senkt dann an der Kontaktfläche zwischen Probenmaterial und Absorber die Konzentration im Probenmaterial auf den Wert 0 herab. Für diese Randbedingungen berechnet sich die vom Absorber pro Flächeneinheit aufgenommene Wasserstoffmenge $M_t$ als Funktion der Zeit t zu

$$M_t = c_0 \sqrt{\frac{Dt}{\pi}} \ .$$

Dabei ist D der Diffusionskoeffizient des Wasserstoffs im Probenmaterial (erstes Material). Dieser Diffusionskoeffizient ist entweder bereits bekannt aus der Literatur oder läßt sich nach bekannten Methoden experimentell für das jeweilige Probenmaterial bestimmen. $M_t$ ist die im Absorber akkumulierte Wasserstoffmenge, die ebenfalls mit verschiedenen Methoden ermittelt werden kann (siehe entsprechende Ausführungen in der Beschreibung). Über die zuletzt aufgeführte Gleichung kann mit Hilfe dieser Werte dann die Wasserstoffkonzentration $C_0$ im Probenmaterial errechnet werden. Die Gleichung gilt jedoch nur so lange, solange an den vom Absorber am weitesten entfernten Probenstellen die Ausgangskonzentration $C_0$ noch nicht abgesenkt wurde. Im vorliegenden Beispiel ist das für Zeiten kleiner als 0,4 $d_A^2/D$ = 4000 s der Fall.

$M_t$ ist dabei nicht nur für die Bestimmung der Konzentration $c_0$ von Bedeutung, sondern darüber hinaus ein interessanter Wert für die Beurteilung, ob ein Probenmaterial eine Wasserstoffmenge enthält, die zu einer Wasserstoffversprödung führt oder führen kann. Dementsprechend kann die Messung bzw. Bestimmung der Größe $M_t$ unter Umständen von größerem Wert sein als die Bestimmung von $c_0$, da z. B. die Anreicherung von Wasserstoff an einem neu gebildeten Riß ebenfalls durch das Produkt $C_0 \sqrt{D}$ bestimmt wird. Somit ist $M_t$ bzw. das daraus berechnete Produkt $C_0 \sqrt{D}$ ein Kennwert für die Neigung des Probenmaterials zur Wasserstoffversprödung.

Überträgt man nun diese Betrachtung auf das oben genannte Beispiel mit den dort genannten Werten für $C_0$ und D, so ergibt sich aus der oben genannten Formel für $M_t$ zunächst

$$M_t = 2{,}2 \cdot 10^{-9} \left[ \frac{Mol-H}{cm^2 \sqrt{s}} \right] \sqrt{t} \ .$$

Die im Absorber durch die dorthin überführte Wasserstoffmenge hervorgerufene Konzentrationsänderung an Wasserstoff $\Delta c$ hängt vom Volumen $V_A$ des Absorbers ab. Verwendet man wie im Beispiel eine Absorberschicht der Dicke d, gemessen in cm, so gilt

$$\Delta c = 2{,}2 \cdot 10^{-9} \left[ \frac{Mol - H}{cm^2 \sqrt{s}} \right] \frac{\sqrt{t}}{d} \ .$$

Mit dem Molvolumen $V_a$ (in cm³/mol) des Absorbers errechnet sich daraus die Konzentrationsänderung des Wasserstoffs im Absorber in at.-% zu

$$\Delta c = \frac{2{,}2 \cdot 10^{-7} V_a \sqrt{t}}{d} at. - \% \ .$$

In der im Beispiel genannten Niobschicht mit den entsprechenden Werten für $V_a$ und d sowie eine Absorptionszeit von t=900 s errechnet sich aus der letzten Gleichung für $\Delta c$ ein Wert von 3,6 at.-%.
Dies entspricht in guter Übereinstimmung dem Wert, der als Konzentrationswert an Wasserstoff in der Niobschicht im Beispiel experimentell (durch elektrochemische Messung) zu $\Delta c$ = 2,4 at.-% bestimmt wird. Diese Übereinstimmung zwischen experimentellem Wert und rechnerischer Ableitung zeigt, daß die in der Berechnung zugrundegelegte Diffusionskonstante von 1E-6 cm²/s plausibel ist.

In der Praxis wird dann so vorgegangen, daß die Wasserstoffkonzentration bzw. Änderung der Wasserstoffkonzentration (gasförmige Verunreinigung) im Absorber (zweites Material, z. B. Niob) quantitativ bestimmt wird, z. B. durch eine elektrochemische Messung oder eine Messung der Röntgenbeugung. Mit Hilfe der aufgeführten Gleichungen und der bekannten Parameter (Diffusionskonstante D von Wasserstoff in der Probe, Dicke d der Absorberschicht und Molvolumen $V_a$) wird dann die (Ausgangs-)Wasserstoffkonzentration $C_0$ in der Probe rechnerisch ermittelt. Auch der entsprechende Kennwert $M_t$ kann so ermittelt werden.

Es versteht sich bei der Erfindung, daß insbesondere die Diffusionszeit (Meßzeit, Versuchszeit) und auch die Dicke der Absorberschicht d in weiten Grenzen variiert und dementsprechend an das jeweilige Probenmaterial und dessen Dimensionen/Volumina angepaßt werden können. So wird es in der Regel so sein, daß bei kleineren Wasserstoffgehalten in der Probe dünnere Absorberschichten zu einem genaueren Ergebnis führen werden, während beispielsweise bei größeren Wasserstoffgehalten in der Probe dickere Absorberschichten sinnvoll sind. Für die im Beispiel genannten Meßmethoden kann die Niobschicht beispielweise bis auf 20 nm Dicke reduziert werden.

Wie bereits in der Beschreibung erläutert, kann das Beispiel auch so modifiziert werden, daß auf dieselbe Stahlprobe verschiedene (zweite) Materialien mit unterschiedlicher Affinität für Wasserstoff als Absorber aufgebracht werden. Dies ermöglicht dann Aussagen, wie stark der in der Probe vorhandene Wasserstoff in der Probe gebunden ist.
In der bisherigen Diskussion wurde ja von einem (ideal gewähltes) Absorber ausgegangen, der also eine sehr hohe Wasserstoffaffinität besitzt und dementsprechend den gesamten in der Probe enthaltenen Wasserstoff aufnimmt. Ein solcher (ideal gewählter) Absorber reduziert die Wasserstoffkonzentration an der Kontaktfläche (Grenzfläche zwischen Probe und Absorber) auf den Wert 0 (siehe obige Formel). Ein weniger starker Absorber mit geringerer Affinität für Wasserstoff (Absorber i) kann diese Reduktion der Wasserstoffkonzentration beispielsweise nur bis zu einem Wert $c_i$ ermöglichen. Dann modifiziert sich die entsprechende oben genannte Gleichung zu

$$M_t = \left( c_0 - c_i \right) \sqrt{\frac{Dt}{\pi}} \ .$$

Damit wird die vom i-ten Absorber aufgenommene Wasserstoffmenge entsprechend reduziert. Ein Vergleich mit dem (idealen) Absorber liefert den Wert $c_i$, der ein Maß für die Bindungsstärke des verbliebenen Anteils ($c_0 - c_i$) an Wasserstoff in der Probe ist.

**Beispiel 2**

Zwei Stahlzylinder (jeweils von den Sorten ST37 und 9S20K) mit einem Durchmesser von 10 mm und einer Höhe von 3 mm wurden in der Mitte einer Stirnfläche mit einem Bohrloch von 0,9 mm Durchmesser und einer Tiefe von 1 mm versehen. In die Bohrlöcher wurde jeweils ein angespitzter Vanadiumdraht mit einem Durchmesser von 1 mm mit Hilfe einer Bohrmaschine unter Rotation eingedrückt (reibverschweißt). Auf den gegenüberliegenden Stirnflächen der Stahlzylinder wurde kathodisch Wasserstoff aus 1 N Schwefelsäure, die mit Arsentrioxid gesättigt war, mit einer Stromdichte von 50 bis 100 mA/cm² abgeschieden. Nach einer Verzögerungszeit von einer Stunde beim ST37-Stahl bzw. mehreren Stunden beim 9S20K-Stahl wurde eine konstante Widerstandsänderung im gegenüberliegenden Vanadiumdraht detektiert. Die Änderung betrug bis zu 1E-3 in einer Stunde, was einer Konzentrationsänderung von 0,02 at.-% im Vanadiumdraht entspricht. Daraus berechnet sich auf der Elektrolytseite des Stahls eine Wasserstoffkonzentration von 2 Gew.-ppm. Eine genauere Berechnung dieser Konzentration bei den tatsächlich vorliegenden Randbedingungen ist über eine numerische Lösung des 2. Fickschen Gesetzes möglich. Bei der Messung des elektrischen Widerstandes des Vanadiumdrahtes wurde ein Strom von 100 mA durch den Draht und einen Vergleichsdraht, der nicht im Kontakt mit dem jeweiligen Stahl verbunden war und somit keinen Wasserstoff aufnehmen konnte, geleitet. Der Spannungsabfall wurde an beiden Drähten über eine Länge von 8 mm mehrmals innerhalb einer Sekunde gemessen. Aus der gemittelten Differenz beider Spannungsabfälle und Normierung auf den gemittelten Spannungsabfall am Vergleichsdraht, konnte so eine durch den Wasserstoff verursachte Widerstandsänderung von weniger als 1 E-3 gemessen werden.

**Patentansprüche**

1.  Verfahren zum Nachweis von Wasserstoff in Stahl als erstem Material, **dadurch gekennzeichnet, dass**

    - der im Stahl vorhandene Wasserstoff durch Diffusion mindestens teilweise in Vanadium, Niob oder Tantal oder deren Legierungen als zweitem Material überführt wird,
    - die Konzentration des Wasserstoffs im zweiten Material quantitativ bestimmt wird, und
    - daraus die Konzentration des Wasserstoffs im ersten Material quantitativ bestimmt wird.

2.  Verfahren nach Anspruch 1, **dadurch gekennzeichnet, dass** die Überführung des Wasserstoffs aus dem ersten Material in das zweite Material unter Anlegen eines elektrischen Feldes erfolgt.

3.  Verfahren nach einem der vorhergehenden Ansprüche, **dadurch gekennzeichnet, dass** das zweite Material in länglicher bzw. langgestreckter Form, insbesondere als Band, Stift oder Draht mit einem Substrat oder einer Probe aus dem ersten Material in Kontakt gebracht ist.

4.  Verfahren nach Anspruch 3, **dadurch gekennzeichnet, dass** das zweite Material mit dem ersten Material durch Verschweißen in Kontakt gebracht ist.

5.  Verfahren nach einem der vorhergehenden Ansprüche, **dadurch gekennzeichnet, dass** das zweite Material als Schicht auf ein Substrat oder eine Probe aus dem ersten Material aufgebracht ist, wobei vorzugsweise die Schichtdicke der Schicht des zweiten Materials weniger als 1 $\mu$m, insbesondere weniger als 250 nm, beträgt.

6.  Verfahren nach Anspruch 5, **dadurch gekennzeichnet, dass** die Schichtdicke des zweiten Materials zwischen 10 und 100 nm, vorzugsweise ca. 20 nm, beträgt.

7.  Verfahren nach einem der vorhergehenden Ansprüche, **dadurch gekennzeichnet, dass** die Anwesenheit des Wasserstoffs in dem zweiten Material durch Messung des elektrischen Widerstands detektiert wird.

8.  Verfahren nach einem der vorhergehenden Ansprüche, **dadurch gekennzeichnet, dass** die pro Flächeneinheit vom zweiten Material aufgenommene Menge an Wasserstoff als Funktion der Zeit berechnet wird und als Kennwert für Eigenschaften des ersten Materials, insbesondere als Kennwert für die Neigung des ersten Materials zur Wasserstoffversprödung gewählt wird.

**Claims**

1.  Method for detecting hydrogen in steel as a first material, **characterized in that**

    - the hydrogen in the steel is transferred by way of diffusion at least partially into vanadium, niobium or tantalum or the alloys thereof as a second material,
    - the concentration of the hydrogen in the second material is quantitatively determined, and
    - on this basis the concentration of the hydrogen in the first material is quantitatively determined.

2.  Method according to Claim 1, **characterized in that** the hydrogen is transferred from the first material into the second material with the application of an electric field.

3.  Method according to one of the preceding claims, **characterized in that** the second material in oblong or elongated form, in particular as a strip, pin or wire, is brought in contact with a substrate or a sample of the first material.

4.  Method according to Claim 3, **characterized in that** the second material is brought in contact with the first material by way of welding.

5.  Method according to one of the preceding claims, **characterized in that** the second material is applied as a layer onto a substrate or a sample of the first material, wherein the layer thickness of the layer of the second material is preferably less than 1 $\mu$m, in particular less than 250 nm.

6.  Method according to Claim 5, **characterized in that** the layer thickness of the second material is between 10 and 100 nm, preferably about 20 nm.

7.  Method according to one of the preceding claims, **characterized in that** the presence of the hydrogen in the second material is detected by way of measuring the electrical resistance.

8.  Method according to one of the preceding claims,

characterized in that the quantity of hydrogen absorbed by the second material per unit area is calculated as a function of time and is chosen as a characteristic value for properties of the first material, in particular as a characteristic value for the tendency of the first material to hydrogen embrittlement.

**Revendications**

1. Procédé de détection de la présence d'hydrogène dans l'acier, constituant un premier matériau, **caractérisé en ce que**

   - au moins une partie de l'hydrogène présent dans l'acier est transférée par diffusion dans du vanadium, du niobium, du tantale ou leurs alliages, constituant un deuxième matériau,
   - la concentration de l'hydrogène dans le deuxième matériau est déterminée quantitativement et
   - la concentration de l'hydrogène dans le premier matériau est déterminée quantitativement à partir de celle-ci.

2. Procédé selon la revendication 1, **caractérisé en ce que** le transfert de l'hydrogène depuis le premier matériau jusque dans le deuxième matériau s'effectue par application d'un champ électrique.

3. Procédé selon l'une des revendications précédentes, **caractérisé en ce que** le deuxième matériau présentant une forme allongée ou étendue, en particulier sous la forme d'un feuillard, d'une tige ou d'un fil, est mis en contact avec un substrat ou un échantillon du deuxième matériau.

4. Procédé selon la revendication 3, **caractérisé en ce que** le deuxième matériau est mis en contact avec le premier matériau par soudage.

5. Procédé selon l'une des revendications précédentes, **caractérisé en ce que** le deuxième matériau est appliqué sur un substrat ou un échantillon du premier matériau sous la forme d'une couche, l'épaisseur de la couche du deuxième matériau étant de préférence inférieure à 1 $\mu$m et en particulier inférieure à 250 nm.

6. Procédé selon la revendication 5, **caractérisé en ce que** l'épaisseur de la couche du deuxième matériau est comprise entre 10 et 100 nm et de préférence est d'environ 20 nm.

7. Procédé selon l'une des revendications précédentes, **caractérisé en ce que** la présence de l'hydrogène dans le deuxième matériau est détectée par mesure de la résistance électrique.

8. Procédé selon l'une des revendications précédentes, **caractérisé en ce que** la quantité d'hydrogène absorbée par unité de surface dans le deuxième matériau est calculée en fonction du temps et est sélectionnée comme valeur caractéristique de propriétés du premier matériau et en particulier comme valeur caractéristique de la tendance du premier matériau à une fragilisation par l'hydrogène.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 3732076 A

- EP 0015428 A1

**In der Beschreibung aufgeführte Nicht-Patentliteratur**

- **HOFFMANN, F. ; LINKEWITZ, T. ; MAYR, P.** Wasserstoffversprödung - Meinungen und Fakten. *Einsatzhärtung. Berichtsband der AWT-ATTT-Tagung,* April 1998, 29, 30
- **AACHEN ; WIESBADEN.** Arbeitsgemeinschaft Wärmebehandlung und Werkstofftechnik e.V. (AWT)/Association Technique de Traitement Thermique (ATTT. *Arbeitsgemeinschaft Wärmebehandlung und Werkstofftechnik e.V. (AWT)/Association Technique de Traitement Thermique (ATTT,* 1998, 259-267
- **HOFFMANN, F. ; LINKEWITZ, T. ; MAYR, P.** Wasserstoffaufnahme beim Einsatzhärten. *HTM,* 1999, vol. 54, 10-13

- Aufkohlen in Gasen. **WYSS, U.** Wärmebehandlung der Bau- und Werkzeugstähle. BAZ Verlag, 1978
- **STRENG, H. ; GROSCH, J. ; RAZIM, C.** Wasserstoffaufnahme und -abgabe beim Einsatzhärten. *HTM,* 1987, vol. 42, 245-260
- **NEUMANN, P.** Grundlagen der Wirkung von Wasserstoff auf die Rißbildung in Stählen. *Stahl u. Eisen,* 1987, vol. 107, 577-583
- **R. KIRCHHEIM.** *Acta Metallurgica,* 1979, vol. 27, 869-878